# EUROPÄISCHE PATENTSCHRIFT

(11) **EP 2 702 849 B1**
(45) Veröffentlichungstag und Bekanntmachung des Hinweises auf die Patenterteilung: **08.04.2015**
(21) Anmeldenummer: 13401088.3
(22) Anmeldetag: 23.08.2013
(51) Int. Cl.: A01C 7/20, A01C 7/08

(54) **Sämaschine**
Sowing machine
Semoir

(30) Priorität: 30.08.2012 DE 102012108006
(43) Veröffentlichungstag der Anmeldung: 05.03.2014
(73) Patentinhaber: Amazonen-Werke H. Dreyer GmbH & Co. KG, 49205 Hasbergen (DE)
(72) Erfinder: Brüggemann, Klaus, 27777 Ganderkesee (DE); Mertens, Daniel, 26135 Oldenburg (DE); Wilken, Martin, 26123 Oldenburg (DE)

(56) Entgegenhaltungen:
- EP-B1- 0 216 057
- FR-A- 1 229 027
- FR-A1- 2 641 441

## Beschreibung

Die Erfindung betrifft eine Sämaschine gemäß des Oberbegriffes des Patentanspruches 1.

Eine derartige Sämaschine ist in EP 0 216 057 B1 beschrieben. Diese Sämaschine weist eine Dosiereinrichtung auf, mittels welcher die einzelnen Saatkörner vereinzelt werden. Die vereinzelten Saatkörner werden in Saatleitungen übergeben, die zu Säscharen führen. Die Säschare ziehen Säfurchen in den Boden, in welche die vereinzelten Saatkörner abgelegt werden. Die von der Dosiereinrichtung zu den Säscharen führenden Saatleitungen werden über eine Druckaufschlagungseinrichtung mit Druckluft beaufschlagt werden, so dass mit einem Förderluftstrom die Saatkörner von der Dosiereinrichtung in den Saatleitungen bis zu den Säscharen gefördert werden. Den Säscharen und den Ausmündungen der Saatleitungen sind auf dem Boden abrollende Saatgutfangrollen zugeordnet. Die Enden der Saatleitungen mit den Ausmündungen sind so ausgerichtet, dass das jeweilige Saatskorn von dem Förderluftstrom in Richtung des von der Saatgutfangrolle und dem Boden gebildeten keilförmigen Bereiches geschleudert und der Saatgutfangrolle gefangen wird.

Diese Anordnung hat sich bewährt. Jedoch bei schwierigen Einsatzverhältnissen, insbesondere auf sehr nassen, klebrigen und schweren Böden kommt es zu Verstopfungen aufgrund von anhaftenden Bodenteilen an den Saatgutfangrollen. Unter diesen schwierigen Verhältnissen musste bisher das Ausbringen von Saatgut unterbrochen werden.

Durch die FR 2 641 441 A1 ist es bekannt, dass vereinzelte Saatgut mittels einer Schwerkraftförderung durch Leitungen dem Boden zuzuführen. Hier fällt das Saatgut allein aufgrund der allein auf das Saatgut einwirkenden Schwerkraft von der Dosiereinrichtung durch die Saatleitung in die Säfurche. Insbesondere bei großen Fallhöhe ist eine reine Schwerkraftförderung nachteilig, weil es hier zu einer Verungleichmäßigung der vereinzelten Saatkörner zwischen der Dosiereinrichtung und den Säscharen, insbesondere bei einer großen Fallhöhe kommt.

Der Erfindung liegt die Aufgabe zu Grunde, die Sämaschine auch bei schwierigen Einsatzverhältnissen universeller einsetzbar zu machen.

Diese Aufgabe wird erfindungsgemäß dadurch gelöst, dass die Druckluftbeaufschlagungseinrichtung über eine Schalteinrichtung zur wahlweisen Förderung der dosierten Saatkörner mittels Druckluft oder ohne Druckluft mittels Schwerkraft in der jeweiligen Saatleitung zu- und/oder abschaltbar ist.

Infolge dieser Maßnahme kann die Sämaschine sowohl mit einer Druckluftförderung wie mit einer Schwerkraftförderung für das Saatgut von der Dosiereinrichtung zum Boden in die Säfurche entsprechend den jeweiligen Einsatzbedingungen einfacher Weise betrieben werden. Bei nassen und/oder klebrigen Einsatzverhältnissen lässt sich dann einfach die Druckluftförderung abschalten und die Saatgutfangrollen ausbauen oder anheben, so dass verstopfungsfrei gearbeitet werden kann.

Um in einfacher Weise die Stärke der Druckluftförderung für die vereinzelten Saatkörner an die jeweiligen Einsatzbedingungen anpassen zu können, ist vorgesehen, dass der Druckluftbeaufschlagungseinrichtung eine Druckregeleinrichtung zugeordnet ist.

Die jeweils gewünschte Förderluftmenge entsprechend den jeweiligen Einsatzbedingungen lässt sich in einfacher Weise dadurch einstellen, dass über der Druckregeleinrichtung der Druckluftbeaufschlagungseinrichtung die der Saatleitung zugeführte Luftmenge veränderbar ist.

Um in einfacher Weise einen Förderluftstrom in den einzelnen Saatleitungen zur Saatgutförderung zu schaffen bzw. den Förderluftstrom in die Saatleitung einzuschleusen, ist vorgesehen, dass im jeweiligen Bereich der Einmündung der Druckluftbeaufschlagungseinrichtung in die jeweilige Saatleitung jeweils eine Strahlpumpe angeordnet ist.

In einfacher Weise ist vorgesehen, dass die Strahlpumpe als Ejektor ausgebildet ist.

In einer Ausführungsform ist vorgesehen, dass die Sämaschine als zumindest ein Vereinzelungsorgan aufweisende Einzelkornsämaschine ausgebildet ist, dass das Vereinzelungsorgan im Drehrichtung beabstandet zueinander mit einem Luftdruckunterschied beaufschlagtbare Perforationen, an denen sich bei der Durchführung durch einen Saatgutvorrat Saatkörner anlagern, aufweist.

Um in sicherer Weise die einzelnen Saatkörner jeweils an der vorgesehenen Stelle und in dem jeweils vorgesehenen Abstand zueinander in der Säfurche platzieren zu können, ist vorgesehen, dass die Ausbringelemente als Säfurchen in den Boden ziehende Säschare ausgebildet sind, dass hinter den Säscharen mit diesen fluchtend angeordnete und drehbar gelagerte Saatgutandruckrollen angeordnet sind, dass das Ende der jeweiligen Saatgutleitung vor der Lauffläche der zugeordneten Saatgutandruckrolle und dem Grund der Säfurche endet und derart ausgrichtet ist, dass die Längsmittellinie des Endes der Saatgutleitung in dem von dem Grund der Säfurche und der unteren vorderen Lauffläche der Saatgutandruckrolle gebildeten Öffnungswinkel weist.

Um eine reibungslose Förderung des Saatgutes bei der Schwerkraftförderung, wenn der Förderluftstrom in der Saatleitung abgeschaltet ist, von der Dosiereinrichtung bis zur Säfurche zu gewährleisten, ist vorgesehen, dass zumindest der untere nach hinten gerichtete Bereich der Saatleitung abnehmbar angeordnet ist.

Um bei schwierigen Einsatzverhältnissen, insbesondere bei nassen und klebrigen Böden, ein verstopten der Ablageeinrichtung der Sämaschine zu vermeiden, und um dennoch eine akzeptable Saatgutablage bei nassen und klebrigen Bedingungen zu erreichen, ist vorgesehen, dass die Saatgutandruckrolle vom Boden der Säfurche abhebbar und/oder abnehmbar an der Säscharhalterung angeordnet ist.

Um unter schwierigen Einsatzbedingungen ein Ausbringen des Saatgutes zu ermöglichen, ist vorgesehen, dass bei nassen Einsatzbedingungen, wenn Boden an die Saatandruckrolle anbackt, über die Schalteinrichtung die Druckluftbeaufschlagungseinrichtung zur Förderung des Saatgutes ohne Druckluft nur mittels Schwerkraft in den jeweiligen Saatleitungen abgeschaltet und die Saatgutandruckrolle vom Boden der Säfurche abgehoben und/oder abgenommen wird.

Um ein verstopfungsfreies Einbringen des Saatgutes auch unter schwierigen Bedingungen zu gewährleisten, ist vorgesehen, dass bei nassen Einsatzbedingungen, wenn Boden an die Saatandruckrolle anbackt, die Saatgutandruckrolle vom Boden der Säfurche abgehoben und/oder abgenommen wird.

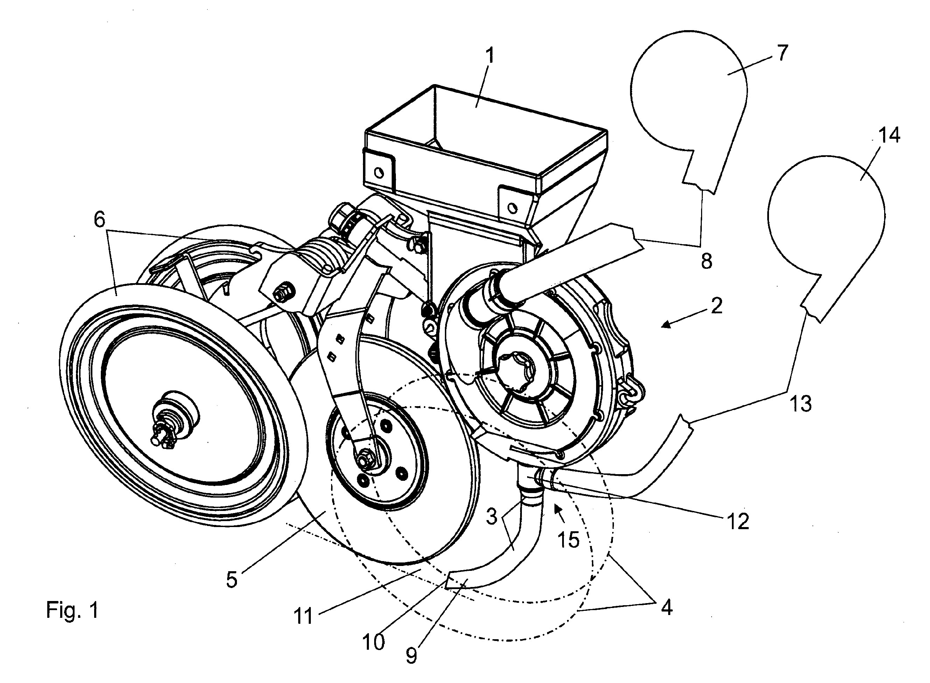

Weitere Einzelheiten der Erfindung sind der Beispielsbeschreibung zu entnehmen. Die Zeichnungen zeigen
- Fig.1: eine Säeinheit einer als Einzelkornsämaschine ausgebildeten Sämaschine mit einem Förderluftstrom zur Druckluftförderung des Saatgutes in der Saatleitung in perspektivischer und Prinzipdarstellung und
- Fig.2: die Säeinheit nach Fig.1 ohne einen Förderluftstrom zur Schwerkraftförderung des Saatgutes in der Saatleitung in gleicher Darstellungsweise.

Die als Einzelkornsämaschine ausgebildete Sämaschine weist einen Vorratsbehälter 1 zur Aufnahme der Saatkörner des auszubringenden Saatgutes auf. Weiterhin weist die Sämaschine zumindest eine mit dem Vorratsbehälter 1 verbundene und angetriebene Dosiereinrichtung 2 auf. Von der jeweiligen Dosiereinrichtung 2 führt eine Saatleitung 3 in Richtung des Bodens bzw. einer von einem mit strichpunktierten Linien dargestellten Säschar 4 in den Boden gezogenen Säfurche. Weiterhin sind der Saatleitung 3 eine Saatgutfangrollen 5, Andrück- und Tiefenführungsrollen 6 sowie das Säschar 4 zugeordnet. Das Säschar 4 ist als Doppelscheibenschar ausgebildet. Zwischen den beiden Scharscheiben des Doppelscheibenschars 4 ist die Saatgutleitung 3 im Bereich der Säfurche ausmündend angeordnet.

Die Dosiereinrichtung 2 des Vereinzelungsaggregates der Einzelkornsämaschine ist als mit einem eine Druckdifferenz an dem Vereinzelungsorgan der Dosiereinrichtung 2 erzeugenden Gebläse 7 über eine Leitung 8 verbunden.

Die an der Dosiereinrichtung angeschlossene Saatleitung3, welche in Richtung der Säfurche führt, ist so angeordnet, dass das Ende 9 der Saatgutleitung 3 bzw. die Ausmündung 10 der Saatgutleitung 3 im keilförmigen Bereich 11, der von dem Boden der Säfurche und der Saaandruckrolle 5 gebildet wird, weist. In der Saatgutleitung 3 ist in der Nähe der Dosiereinrichtung 2 eine Strahlpumpe 12 angeordnet, welche über eine Leitung 13 mit einem Druckluftgebläse 14 verbunden ist. Hierdurch wird ein Förderluftstrom durch die in die Saatleitung 3 über die Strahlpumpe 11 eingeleitete Druckluft erzeugt, so dass die von der Dosiereinrichtung 2 in dieser Saatgutleitung 3 eingespeisten und vereinzelten Samenkörner von den Förderluftstrom bis zum Ende 9 der Saatgutleitung 3 gefördert werden. Am Ausmündungsende 10 der Saatgutleitung 3 werden die Saatkörner im Richtung des von der Säfurche und der Saatandrückrolle 5 gebildeten keilförmigen Bereich 11 geschleudert und von der Saatandruckrolle 5 eingeklemmt und in den Boden der Säfurche gedrückt.

Hierbei bilden das Druckgebläse 13 und die Strahlpumpe 11 die Druckluftbeaufschlagungseinrichtung. Mittels einer nicht dargestellten Schalteinrichtung kann die Druckluftzufuhr zu der Strahlpumpe 12 von dem Druckluftgebläse 13 zu der zur Säfurche führenden Saatleitung 3 zu- oder abgeschaltet werden. Unterzu- und abschalten über die Schalteinrichtung wird auch verstanden, dass die Strahlpumpe 12 und/oder die Druckluftzuführung 13, 14 von der Dosiereinrichtung 2 bzw. der Saatleitung 3 abgenommen wird.

Um die Förderluftmenge an die jeweiligen Einsatzverhältnisse anpassen zu können, ist eine nicht dargestellte Druckregeleinrichtung vorgesehen, die die zugeführte Luftmenge über die Strahlpumpe 12 in die Saatleitung 3 entsprechend einstellbar macht.

Zur Einmündung der von dem Druckgebläse 14 kommenden Druckluftleitung 12 und der zugeordneten Strahlpumpe 12 ist in der Saatleitung 3 ein Zweigstück 15 angeordnet.

Die Strahlpumpe 12 ist in vorteilhafter Weise als Ejektor ausgebildet.

Das Vereinzelungsorgan der Dosiereinrichtung 2 der Einzelkornsämaschine weist in nicht dargestellter Weise in Drehrichtung beabstandet zueinander mit einem Luftdruckunterschied beaufschlagte Perforationen, an denen sich bei Durchführung durch einen Saatgutvorrat aus dem Vorratsbehälter 1 Saatkörner anlagern, auf.

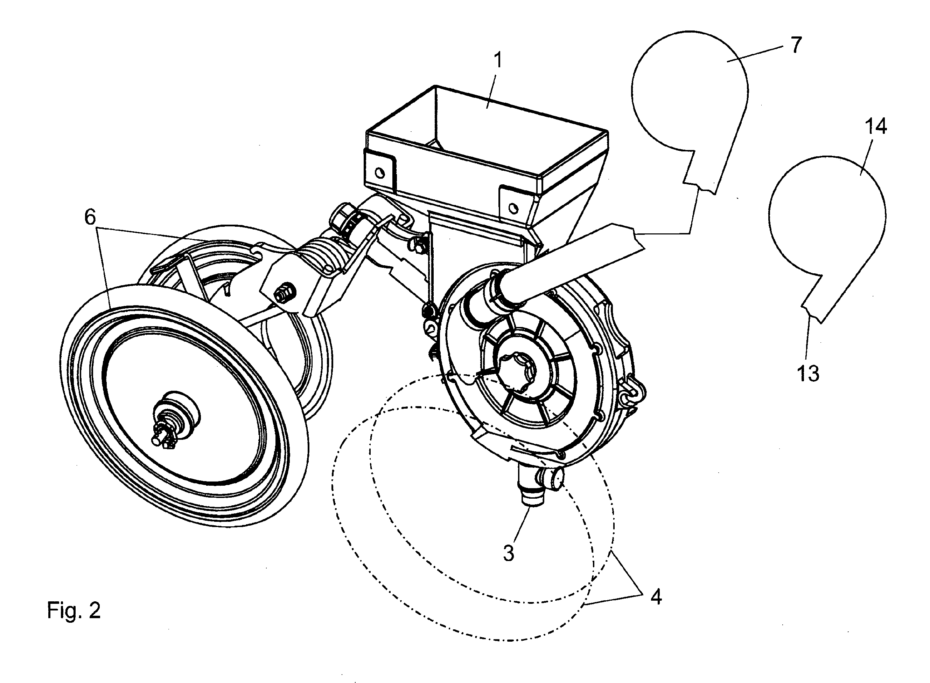

Über die Zu- und Abschaltungsmöglichkeit der Zuführung der der Förderluftstrom über die Schalteinrichtung kann die Sämaschine auch ohne Förderluftstrom in der Saatleitung 3 zur Förderung des vereinzelten Saatgutes entsprechend der Ausgestaltung gemäß Fig.2 eingesetzt werden. Für diesen Einsatzfall, in dem das Saatgut nur mittels Schwerkraft zum Boden gefördert wird, wird der untere Teil und nach hinten gerichtete Teil 8 der Saatleitung 3 abgenommen. Anstelle dieses abgenommenen und gekrümmt ausgeführten Bereich der Saatleitung 3 kann in nicht dargestellter Weise auch ein geradlinig oder schräg nach unten ausgerichtetes Saatleitungstück angeschlossen werden. Dieses geschieht dann, wenn die Sämaschine auf nassen, klebrigen und schweren Böden eingesetzt wird, d. h., wenn an der Saatgutfangrolle 5 zu viele Bodenteile anhaften. In diesem Falle wird auch, wie Fig.2 zeigt die Saatgutfangrolle 5 abgenommen.

Somit kann also in leicht ersichtlicherweise die Druckluftbeaufschlagungseinrichtung 12 über eine Schalteinrichtung zur wahlweisen Förderung der dosierten Saatkörner mittels Druckluft oder ohne Druckluft nur mittels Schwerkraft in der jeweiligen Saatgutleitung 3 zu- und/oder abgeschaltet werden. Wie bereits erwähnt, wird hierunter auch verstanden, dass die Druckluftzufuhr- bzw. die die Druckluft zuführenden Elemente 13 von der Saatgutleitung 3 bzw. Sämaschine abgenommen werden.

Zusammenfassend wird auf folgendes verwiesen:

Die Ausbringlemente sind als Säfurchen in den Boden ziehende Säschare 4 ausgebildet. Hinter den Säscharen 4 sind mit diesen fluchtend angeordnete und drehbar gelagerte Saatgutandruckrollen 5 angeordnet. Das Ende 9 der jeweiligen Saatgutleitung 3 endet vor der Lauffläche der zugeordneten Saatgutandruckrolle 5 und dem Grund der Säfurche und ist derart ausgerichtet ist, dass die Längsmittellinie des Endes 9, 10 der Saatgutleitung 3 in dem von dem Grund der Säfurche und der unteren vorderen Lauffläche der Saatgutandruckrolle 5 gebildeten Öffnungswinkel 10 weist.

Die Sämaschine lässt sich so betreiben, dass bei nassen Einsatzbedingungen, wenn Boden an die Saatandruckrolle 5 anbackt, über die Schalteinrichtung die Druckluftbeaufschlagungseinrichtung 13, 14 zur Förderung des Saatgutes ohne Druckluft nur mittels Schwerkraft in den jeweiligen Saatleitungen 3 abgeschaltet und die Saatgutandruckrolle 5 vom Boden der Säfurche abgehoben und/oder abgenommen (Fig. 2) wird. Hierbei wird bei nassen Einsatzbedingungen, wenn Boden an die Saatandruckrolle 5 anbackt, die Saatgutandruckrolle 5 vom Boden der Säfurche abgehoben und/oder abgenommen (Fig. 2) wird.

## Patentansprüche

1. Sämaschine mit zumindest einem Vorratsbehälter, zumindest einer angetriebenen Dosiereinrichtung und von der jeweiligen Dosiereinrichtung zu Ausbringelementen führenden Saatleitungen, wobei zwischen der jeweiligen Dosiereinrichtung und der jeweiligen Saatleitung eine Druckluftbeaufschlagungseinrichtung zur Förderung der dosierten Saatkörner in der jeweiligen Saatleitung einmündet, **dadurch gekennzeichnet, dass** die Druckluftbeaufschlagungseinrichtung (13, 14) über eine Schalteinrichtung zur wahlweisen Förderung der dosierten Saatkörner mittels Druckluft oder ohne Druckluft mittels Schwerkraft in der jeweiligen Saatleitung (3) zu- und/oder abschaltbar ist.

2. Sämaschine nach Anspruch 1, **dadurch gekennzeichnet, dass** der Druckluftbeaufschlagungseinrichtung (13, 14) eine Druckregeleinrichtung zugeordnet ist.

3. Sämaschine nach Anspruch 2, **dadurch gekennzeichnet, dass** über der Druckregeleinrichtung der Druckluftbeaufschlagungseinrichtung (13, 14) die der Saatleitung (3) zugeführte Luftmenge veränderbar ist.

4. Sämaschine nach Anspruch 1, **dadurch gekennzeichnet, dass** im jeweiligen Bereich der Einmündung (15) der Druckluftbeaufschlagungseinrichtung (13) in die jeweilige Saatleitung (3) jeweils eine Strahlpumpe (12) angeordnet ist.

5. Sämaschine nach Anspruch 1, **dadurch gekennzeichnet, dass** die Strahlpumpe (12) als Ejektor ausgebildet ist.

6. Sämaschine nach zumindest einem der vorstehenden Ansprüche, **dadurch gekennzeichnet, dass** die Sämaschine als zumindest ein Vereinzelungsorgan (2) aufweisende Einzelkornsämaschine ausgebildet ist, dass das Vereinzelungsorgan (2) im Drehrichtung beabstandet zueinander mit einem Luftdruckunterschied beaufschlagtbare Perforationen, an denen sich bei der Durchführung durch einen Saatgutvorrat Saatkörner anlagern, aufweist.

7. Sämaschine nach zumindest einem der vorstehenden Ansprüche, **dadurch gekennzeichnet, dass** die Ausbringlemente als Säfurchen in den Boden ziehende Säschare (4) ausgebildet sind, dass hinter den Säscharen (4) mit diesen fluchtend angeordnete und drehbar gelagerte Saatgutandruckrollen (5) angeordnet sind, dass das Ende (9, 10) der jeweiligen Saatgutleitung (3) vor der Lauffläche der zugeordneten Saatgutandruckrolle (5) und dem Grund der Säfurche endet und derart ausgrichtet ist, dass die Längsmittellinie des Endes der Saatgutleitung (3) in dem von dem Grund der Säfurche und der unteren vorderen Lauffläche der Saatgutandruckrolle gebildeten Öffnungswinkel (11) weist.

8. Sämaschine nach zumindest einem der vorstehenden Ansprüche, **dadurch gekennzeichnet, dass** zumindest der untere nach hinten gerichtete Bereich (9) der Saatleitung (3) abnehmbar angeordnet ist.

9. Sämaschine nach zumindest einem der vorstehenden Ansprüche, **dadurch gekennzeichnet, dass** die Saatgutandruckrolle (5) vom Boden der Säfurche abhebbar und/oder abnehmbar an der Säscharhalterung angeordnet ist.

10. Verfahren zum Betreiben einer Sämaschine nach zumindest einem der vorstehenden Ansprüche, **dadurch gekennzeichnet, dass** bei nassen Einsatzbedingungen, wenn Boden an die Saatandruckrolle (5) anbackt, über die Schalteinrichtung die Druckluftbeaufschlagungseinrichtung (13,14) zur Förderung des Saatgutes ohne Druckluft nur mittels Schwerkraft in den jeweiligen Saatleitungen (3) abgeschaltet und die Saatgutandruckrolle (5) vom Boden der Säfurche abgehoben und/oder abgenommen wird.

11. Verfahren zum Betreiben einer Sämaschine nach zumindest einem der vorstehenden Ansprüche, **dadurch gekennzeichnet, dass** bei nassen Einsatzbedingungen, wenn Boden an die Saatandruckrolle (5) anbackt, die Saatgutandruckrolle (5) vom Boden der Säfurche abgehoben und/oder abgenommen wird.

## Claims

1. Sowing machine with at least one storage container, at least one driven metering device and seed lines leading from the respective metering device to dispensing elements, wherein a compressed air application device for conveying the metered seed grains in the respective seed line opens between the respective metering device and the respective seed line, **characterized in that** the compressed air application device (13, 14) can be switched on and/or off via a switching device for optionally conveying the metered seed grains in the respective seed line (3) by means of compressed air or by means of gravity without compressed air.

2. Sowing machine according to Claim 1, **characterized in that** the compressed air application device (13, 14) is assigned a pressure-regulating device.

3. Sowing machine according to Claim 2, **characterized in that** the quantity of air supplied to the seed line (3) is changeable via the pressure-regulating device of the compressed air application device (13, 14).

4. Sowing machine according to Claim 1, **characterized in that** a jet pump (12) is arranged in each case in the respective region of the opening (15) of the compressed air application device (13) into the respective seed line (3).

5. Sowing machine according to Claim 1, **characterized in that** the jet pump (12) is in the form of an ejector.

6. Sowing machine according to at least one of the preceding claims, **characterized in that** the sowing machine is in the form of a single grain sowing machine having at least one singulating member (2), **in that** the singulating member (2) has perforations which, spaced apart from one another in the direction of rotation, can be acted upon by a difference in air pressure and at which seed grains accumulate during the passage through a seed store.

7. Sowing machine according to at least one of the preceding claims, **characterized in that** the dispensing elements are designed as sowing coulters (4) ploughing sowing furrows in the soil, **in that** seed pressing rollers (5) which are arranged in alignment with the sowing coulters (4) and are mounted rotatably are arranged behind said sowing coulters (4), **in that** the end (9, 10) of the respective seed line (3) ends upstream of the running surface of the assigned seed pressing roller (5) and the base of the sowing furrow and is oriented in such a manner that the longitudinal centre line of the end of the seed line (3) points into the opening angle (11) formed by the base of the sowing furrow and the lower front running surface of the seed pressing roller.

8. Sowing machine according to at least one of the preceding claims, **characterized in that** at least the lower, rearwardly directed region (9) of the seed line (3) is arranged removably.

9. Sowing machine according to at least one of the preceding claims, **characterized in that** the seed pressing roller (5) is arranged on the sowing coulter holder so as to be raisable from the soil of the sowing furrow and/or so as to be removable.

10. Method for operating a sowing machine according to at least one of the preceding claims, **characterized in that**, in the event of wet use conditions, when soil sticks onto the seed pressing roller (5), the compressed air application device (13, 14) is switched off via the switching device for conveying the seed only by means of gravity without compressed air in the respective seed lines (3) and the seed pressing roller (5) is raised from the soil of the sowing furrow and/or removed.

11. Method for operating a sowing machine according to at least one of the preceding claims, **characterized in that**, in the event of wet use conditions, when soil sticks onto the seed pressing roller (5), the seed pressing roller (5) is raised from the soil of the sowing furrow and/or removed.

## Revendications

1. Semoir comprenant au moins un réservoir de stockage, au moins un dispositif de dosage entraîné et des conduites de semences menant du dispositif de dosage respectif jusqu'à des éléments d'épandage, un dispositif de sollicitation à air comprimé pour le transport des graines de semences dosées dans la conduite de semences respective débouchant entre le dispositif de dosage respectif et la conduite de semences respective, **caractérisé en ce que** le dispositif de sollicitation à air comprimé (13, 14) peut être activé et/ou désactivé par le biais d'un dispositif de commutation pour transporter de manière sélective les graines de semences dosées au moyen d'air comprimé ou sans air comprimé au moyen de la pesanteur dans la conduite de semences (3) respective.

2. Semoir selon la revendication 1, **caractérisé en ce qu'**un dispositif de régulation de pression est associé au dispositif de sollicitation à air comprimé (13, 14).

3. Semoir selon la revendication 2, **caractérisé en ce que**, par le biais du dispositif de régulation de pression du dispositif de sollicitation à air comprimé (13, 14), la quantité d'air acheminée jusqu'à la conduite de semences (3) peut être modifiée.

4. Semoir selon la revendication 1, **caractérisé en ce qu'**une pompe à jet (12) respective est disposée dans la région respective de l'embouchure (15) du dispositif de sollicitation à air comprimé (13) dans la conduite de semences (3) respective.

5. Semoir selon la revendication 1, **caractérisé en ce que** la pompe à jet (12) est réalisée sous forme d'éjecteur.

6. Semoir selon au moins l'une quelconque des revendications précédentes, **caractérisé en ce que** le semoir est réalisé sous forme de semoir monograine comprenant au moins un organe de séparation (2), **en ce que** l'organe de séparation (2) comprend des perforations espacées les unes des autres dans le sens de rotation et pouvant être sollicitées par une différence de pression d'air, au niveau desquelles perforations des graines de semences s'accumulent lors du passage à travers un réservoir de semences.

7. Semoir selon au moins l'une quelconque des revendications précédentes, **caractérisé en ce que** les éléments d'épandage sont réalisés sous forme de socs de semoir (4) formant des sillons de semis dans le sol, **en ce que** derrière les socs de semoir (4) sont disposés des rouleaux presseurs de semences (5) disposés en alignement avec ceux-ci et montés à rotation, **en ce que** l'extrémité (9, 10) de la conduite de semences (3) respective se termine devant la surface de roulement du rouleau presseur de semences (5) associé et la base du sillon de semis et est orientée de telle sorte que la ligne médiane longitudinale de l'extrémité de la conduite de semences (3) soit tournée dans l'angle d'ouverture (11) formé par la base du sillon de semi et la surface de roulement avant inférieure du rouleau presseur de semences.

8. Semoir selon au moins l'une quelconque des revendications précédentes, **caractérisé en ce qu'**au moins la région (9) inférieure orientée vers l'arrière de la conduite de semences (3) est disposée de manière amovible.

9. Semoir selon au moins l'une quelconque des revendications précédentes, **caractérisé en ce que** le rouleau presseur de semences (5) peut être soulevé de la base des sillons de semis et/ou est disposé de manière amovible sur le support de soc de semoir.

10. Procédé de fonctionnement d'un semoir selon au moins l'une quelconque des revendications précédentes, **caractérisé en ce qu'**en conditions d'utilisation humides, lorsque le sol adhère au rouleau presseur de semences (5), le dispositif de sollicitation à air comprimé (13, 14) est désactivé par le biais du dispositif de commutation pour transporter des semences sans air comprimé et seulement au moyen de la pesanteur dans les conduites de semences (3) respectives et le rouleau presseur de semences (5) est soulevé de la base du sillon de semis et/ou enlevé.

11. Procédé de fonctionnement d'un semoir selon au moins l'une quelconque des revendications précédentes, **caractérisé en ce qu'**en conditions d'utilisation humides, lorsque le sol adhère au rouleau presseur de semences (5), le rouleau presseur de semences (5) est soulevé de la base du sillon de semis et/ou enlevé.
